# EUROPÄISCHE PATENTSCHRIFT

(11) **EP 0 459 244 B1**
(45) Veröffentlichungstag und Bekanntmachung des Hinweises auf die Patenterteilung: **28.02.1996**
(21) Anmeldenummer: 91108011.7
(22) Anmeldetag: 17.05.1991
(51) Int. Cl.: C07D 261/18, C07D 271/06, C07D 263/34, C07D 277/56, C07D 413/12, C07D 417/12, A01N 43/76, A01N 43/74, A01N 43/78, A01N 43/82

(54) **Sulfonylierte Carbonsäureamide**
Sulphonilised carboxylic acid amides
Amides sulfonylés d'acides carboxyliques

(30) Priorität: 30.05.1990 DE 4017338
(43) Veröffentlichungstag der Anmeldung: 04.12.1991
(73) Patentinhaber: BAYER AG, D-51368 Leverkusen (DE)
(72) Erfinder: Wolf, Hilmar, Dr., W-4018 Langenfeld (DE); Kirsten, Rolf, Dr., W-4019 Monheim (DE); Santel, Hans-Joachim, Dr., W-5090 Leverkusen 1 (DE); Lürssen, Klaus, Dr., W-5060 Bergisch Gladbach 2 (DE); Schmidt, Robert R., Dr., W-5060 Bergisch Gladbach 2 (DE)

(56) Entgegenhaltungen:
- EP-A- 0 244 166
- DE-A- 2 610 527
- DE-A- 3 444 918
- US-A- 4 838 925
- CHEMICAL ABSTRACTS, Band 81, Nr. 21, 25 Nov. 1974, Columbus, Ohio, USA; UMEZAWA, HAMAO et al.: "Bleomycins", Seite 454, Spalte 2, Zusammenfassung-Nr. 136 460e
- CHEMICAL ABSTRACTS, Band 95, Nr. 3, 20 Juli 1981, Columbus, Ohio, USA; MEDICI, ALESSANDRO et al.: "Functionalization of thiazoles. Selectivity in the reactions 2-(dimethyl-amino)-1,3-thiazoles with electrophiles: formation of Michael-type adducts and thaizolium salts", Seite 684, Spalte 1, Zusammenfassung-Nr. 24 897k.
- CHEMICAL ABSTRACTS, Band 94, Nr. 9, 2 März 1981, Columbus, Ohio, USA; CHUPP, JHON P. et al.: "Heterocycles from substituted amides. VIII. Oxazole derivatives from reaction of isocyanates with 2-isocyano-acetamides", Seite 713, Spalte 2, Zusammenfassung-Nr. 65 514f.
- CHEMICAL ABSTRACTS, Band 94, Nr. 3, 19 Jänner 1981, Columbus, Ohio, USA; PETYUNIN G.P.: "Synthesis of 5-substituted 1,3,4-oxadiazole-2-carboxylic acid N-(p-toluenesulfonyl)amides", Seit 435, Spalte 1, Zusammenfassung-Nr. 15 648g.

## Beschreibung

Die Erfindung betrifft sulfonylierte Carbonsäureamide, Verfahren zu ihrer Herstellung und ihre Verwendung als Herbizide.

2-Dimethylamino-N-(4-methyl-phenylsulfonyl)-5-thiazolcarboxamid ist bereits als Beispiel für sulfonylierte Carbonsäureamide aus der Literatur bekannt (vgl. J. Org. Chem. 46 (1981), 2790 - 2793). Über die biologischen Eigenschaften dieser Verbindung ist jedoch nichts bekanntgeworden. Desweitern werden in Farm.Zh. (Kiev)1980, (4), 65 (Ukrain) und im J. Heterocycl. Chem. 1980,17(4), 711-15 sulfonylierte Carbonsäureamide beschrieben. Es wurden nun die sulfonylierten Carbonsäureamide der allgemeinen Formel (I)

R¹-CO-NH-SO₂-(A)ₙ-R² (I)

in welcher
- n: für die Zahl 0 steht. Falls n für die Zahl 1 steht, steht A insbesondere für CH₂,
- A: für Sauerstoff, Imino (NH) oder Methylen (CH₂) steht,
- R¹: für einen fünfgliedrigen Heteroarylrest aus der Reihe Oxazolyl, Isoxazolyl, Thiazolyl, Isothiazolyl, Oxadiazolyl, Thiadiazolyl steht, welcher gegebenenfalls einfach oder mehrfach, insbesondere einfach oder zweifach, gleich oder verschieden durch
Halogen oder durch gegebenenfalls halogen-substituiertes C₁-C₆-Alkyl, Phenyl, C₁-C₆-Alkoxy, C₁-C₆-Alkylthio, C₁-C₆-Alkylsulfinyl, C₁-C₆-Alkylsulfonyl, C₁-C₆-Alkylamino oder Di-(C₁-C₄-alkyl)-amino substituiert ist, und
- R²: für den Rest 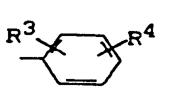 steht, wobei
- R³: für Wasserstoff, Fluor, Chlor, Brom, Iod, Cyano, Nitro, Carboxy oder C₁-C₆-Alkyl stehen, welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist, weiterhin R³
für C₂-C₆-Alkenyl steht , welches gegebenenfalls durch Fluor, Chlor, Brom, Cyano, C₁-C₄-Alkoxycarbonyl, Carboxy oder Phenyl substituiert ist, ferner R³
für C₂-C₆-Alkinyl, welches gegebenenfalls durch Fluor, Chlor, Brom, Cyano, C₁-C₄-Alkoxy-carbonyl, Carboxy oder Phenyl substituiert ist; für C₁-C₄-Alkoxy, welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist; für C₁-C₄-Alkylthio, welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist; für C₃-C₆-Alkenyloxy, welches gegebenenfalls durch Fluor, Chlor, Brom, Cyano oder C₁-C₄-Alkoxycarbonyl substituiert ist, für C₂-C₆-Alkenylthio, welches gegebenenfalls durch Fluor, Chlor, Brom, Cyano, Nitro, C₁-C₃-Alkylthio oder C₁-C₄-Alkoxycarbonyl substituiert ist; C₃-C₆-Alkinyloxy, C₃-C₆-Alkinylthio oder für den Rest -S(O)ₚ-R⁵ steht ,
- R³: weiterhin für Phenyl oder Phenoxy, für C₁-C₄-Alkylcarbonylamino, C₁-C₄-Alkoxycarbonylamino, C₁-C₄-Alkylamino-carbonylamino, Di-(C₁-C₄-alkyl)-amino-carbonylamino, oder für den Rest -CO-R⁶ steht , R³ weiterhin für C₁-C₄-Alkylsulfonyloxy, Di-(C₁-C₄-alkyl)-aminosulfonylamino oder für den Rest -CH=N-R⁷ steht , wobei
- p: für die Zahlen 1 oder 2 steht und
- R⁵: für C₁-C₄-Alkyl steht, welches gegebenenfalls durch Fluor, Chlor, Brom, Cyano oder C₁-C₄-Alkoxy-carbonyl substituiert ist oder R⁵ für C₁-C₄-Alkoxy, C₁-C₄-Alkoxy-C₁-C₄-alkylamino oder Di-(C₁-C₄-alkyl)-amino steht,
- R⁶: für C₁-C₆-Alkyl, C₁-C₆-Alkoxy, welches gegebenenfalls durch Fluor, Chlor, Methoxy oder Ethoxy substituiert ist; C₃-C₆-Cycloalkoxy, C₃-C₆-Alkenyloxy, C₁-C₄-Alkylthio, Amino, C₁-C₄-Alkylamino, C₁-C₄-Alkoxyamino, C₁-C₄-Alkoxy-C₁-C₄-alkyl-amino oder Di-(C₁-C₄-alkyl)-amino steht, welche gegebenenfalls durch Fluor und/oder Chlor substituiert sind und
- R⁷: für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/ oder Chlor substituiertes C₁-C₆-Alkoxy, C₃-C₆-Alkenyloxy, C₃-C₆-Alkinyloxy oder Benzyloxy, R⁷ weiterhin für Amino, C₁-C₄-Alkylamino, Di-(C₁-C₄-alkyl)-amino, Phenylamino, C₁-C₄-Alkyl-carbonylamino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht und
- R⁴: für Wasserstoff steht, sowie Salze von Verbindungen der Formel (I), wobei R³ in ortho-Position steht .

Man erhält die neuen sulfonylierten Carbonsäureamide der Formel (I), wenn man
(a) Carbonsäureamide der allgemeinen Formel (II)

   R¹-CO-NH₂ (II)

   in welcher
   - R¹: die oben angegebene Bedeutung hat,
   mit Sulfonylierungsmitteln der allgemeinen Formel (III)

   X-SO₂-(A)ₙ-R² (III)

   in welcher
   n, A und R² die oben angegebene Bedeutung haben und
   - X: für Halogen steht,
   gegebenenfalls in Gegenwart eines Säureakzeptors und gegebenenfalls in Gegenwart eines Verdünnungsmittels umsetzt, oder wenn man
(b) Carbonsäuren der allgemeinen Formel (IV)

   R¹-COOH (IV)

   in welcher
   - R¹: die oben angegebene Bedeutung hat,
   oder reaktionsfähige Derivate der Carbonsäuren der Formel (IV)
   mit Aminosulfonylverbindungen der allgemeinen Formel (V)

   H₂N-SO₂-(A)ₙ-R² (V)

   in welcher
   n, A und R² die oben angegebene Bedeutung haben,
   oder mit reaktionsfähigen Derivaten der Verbindungen der Formel (V)
   gegebenenfalls in Gegenwart von Reaktionshilfsmitteln und gegebenenfalls in Gegenwart von Verdünnungsmitteln umsetzt und gegebenenfalls die nach Verfahren (a) oder (b) erhaltenen Produkte nach üblichen Methoden in Salze überführt.

Die neuen sulfonylierten Carbonsäureamide der allgemeinen Formel (I) zeichnen sich durch starke herbizide Wirksamkeit aus.

Gegenstand der Erfindung sind vorzugsweise sulfonylierte Carbonsäureamide der Formel (I), in welcher
- n: für die Zahl 0 steht. Falls n für die Zahl 1 steht, steht A insbesondere für CH₂,
- A: für Sauerstoff, Imino (NH) oder Methylen (CH₂) steht,
- R¹: für einen fünfgliedrigen Heteroarylrest aus der Reihe Oxazolyl, Isoxazolyl, Thiazolyl, 1,2,4-Oxadiazolyl, 1,3,4-Oxadiazolyl, 1,2,4-Thiadiazolyl, 1,3,4-Thiadiazolyl steht, welcher gegebenenfalls durch Fluor, Chlor, Brom, Methyl, Ethyl, Propyl, Isopropyl, Butyl, Isobutyl, sec-Butyl, tert-Butyl, Fluormethyl, Chlormethyl, Difluormethyl, Dichlormethyl, Trifluormethyl, Trichlormethyl, Chlorfluormethyl, Chlordifluormethyl, Fluordichlormethyl, Phenyl, Methoxy, Ethoxy, Propoxy, Isopropyloxy, Difluormethoxy, Trifluormethoxy, Chlordifluormethoxy, Methylthio, Ethylthio, Propylthio, Isopropylthio, Difluormethylthio, Trifluormethylthio, Chlordifluormethylthio, Methylsulfinyl, Ethylsulfinyl, Propylsulfinyl, Methylsulfonyl, Ethylsulfonyl, Propylsulfonyl, Methylamino, Ethylamino, Propylamino, Isopropylamino und/oder Dimethylamino substituiert ist, und
- R²: für den Rest 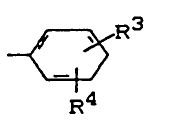 steht, worin
- R³: für Wasserstoff, Fluor, Chlor, Brom, Cyano, Nitro, Carboxy, Methyl, Trifluormethyl, Methoxy, Ethoxy, 2-Chlor-ethoxy, Difluormethoxy, Trifluormethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
- R⁴: für Wasserstoff, steht; wobei R³ in ortho-Position steht .

Die Erfindung betrifft weiter vorzugsweise Salze von Verbindungen der Formel (I) mit Basen, wie z.B. Natrium-, Kalium- oder Calcium-hydroxid, -hydrid, -amid oder -carbonat, Natrium- oder Kalium-C₁-C₄-alkanolaten, Ammoniak, C₁-C₄-Alkylaminen, Di-(C₁-C₄-alkyl)-aminen oder Tri-(C₁-C₄-alkyl)-aminen.

Verwendet man beim erfindungsgemäßen Verfahren (a) beispielsweise 2-Trifluormethyl-oxazol-4-carbonsäureamid und 2-Cyano-phenylmethansulfonsäurechlorid als Ausgangsstoffe, so kann der Reaktionsablauf durch das folgende Formelschema skizziert werden:

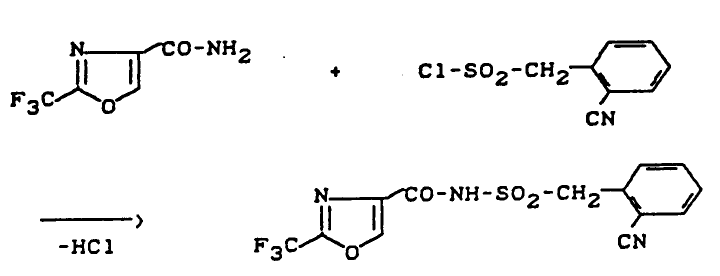

Die beim erfindungsgemäßen Verfahren (a) zur Herstellung von Verbindungen der Formel (I) als Ausgangsstoffe zu verwendenden Carbonsäureamide sind durch die Formel (II) allgemein definiert.

In Formel (II) hat R¹ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R¹ angegeben wurde.

Als Beispiele für die Ausgangsstoffe der Formel (II) seien genannt:
5-Methyl-, 5-Ethyl-, 5-Propyl-, 5-Isopropyl-, 5-Butyl-, 5-Isobutyl-, 5-sec-Butyl- und 5-tert-Butyl-isoxazol-3-carbonsäureamid, 2-Methyl-, 2-Ethyl-, 2-Propyl-, 2-Isopropyl-, 2-Butyl-, 2-Isobutyl-, 2-sec-Butyl- und 2-tert-Butyl-oxazol-4-carbonsäureamid, 5-Methyl-, 5-Ethyl-, 5-Propyl-, 5-Isopropyl-, 5-Butyl-, 5-Isobutyl-, 5-sec-Butyl- und 5-tert-Butyl-1,2,4-oxadiazol-3-carbonsäureamid, 2-Chlor-, 2-Methyl-, 2-Ethyl-, 2-Propyl-, 2-Isopropyl-, 2-Butyl-, 2-Isobutyl-, 2-sec-Butyl, 2-tert-Butyl-, 2-Trifluormethyl-, 2-Methoxy-, 2-Ethoxy-, 2-Propoxy- und 2-Isopropoxy-thiazol-5-carbonsäureamid 2,4-Dichlor-thiazol-5-carbonsäureamid, 2-Methyl-4-chlor-thiazol-5-carbonsäureamid, 2-Methoxy-4-chlor-thiazol-5-carbonsäureamid, 4-Methyl-, 4-Ethyl-, 4-Propyl-, 4-Isopropyl-, 4-Butyl-, 4-Isobutyl-, 4-sec-Butyl- und 4-tert-Butyl-thiazol-2-carbonsäureamid, 5-Methyl-, 5-Ethyl-, 5-Propyl-, 5-Isopropyl-, 5-Butyl-, 5-Isobutyl-, 5-sec-Butyl- und 5-tert-Butyl-thiazol-2-carbonsäureamid.

Die Carbonsäureamide der Formel (II) sind bekannt und/oder können nach an sich bekannten Verfahren hergestellt werden (vgl. RO-P 67530 zit. in Chem. Abstracts 95, 7258k; Helv. Chim. Acta 27 (1944), 1437 - 1438; Chem. Ber. 73 (1940), 1240 - 1252; J. Prakt. Chem. 314 (1972), 447 - 454).

Die beim erfindungsgemäßen Verfahren (a) weiter als Ausgangsstoffe benötigten Sulfonylierungsmittel sind durch die Formel (III) allgemein definiert.

In Formel (III) haben n, A und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, A und R² angegeben wurden und
- X: steht vorzugsweise für Fluor, Chlor oder Brom, insbesondere für Chlor.

Als Beispiele für die Ausgangsstoffe der Formel (III) seien genannt:
Benzolsulfonsäurechlorid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Difluormethoxy-, 2-Trifluormethoxy-, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylamino-carbonyl- und 2-Diethylaminocarbonyl-benzolsulfonsäurechlorid sowie (2-Chlor-phenyl)-, (2-Cyano-phenyl)-, (2-Methoxycarbonyl-phenyl)- und (2-Trifluormethoxy-phenyl)-methansulfonsäurechlorid, 2-Chlor-6-methyl-benzolsulfonsäurechlorid und 2,6-Dichlor-benzolsulfonsäurechlorid.

Die Sulfonylierungsmittel der Formel (III) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid und Pyridin.

Als Säureakzeptoren können bei dem erfindungsgemäßen Vorfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalimetallhydride wie z.B. Natrium- und Kaliumhydrid, Erdalkalimetallhydride wie z.B. Calciumhydrid, Alkalicarbonate und -alkoholate wie Natrium- und Kaliumcarbonat, Natrium- und Kalium-tert.-butylat, ferner aliphatische, aromatische oder heterocyclische Amine, beispielsweise Triethylamin, Trimethylamin, Dimethylanilin, Dimethylbenzylamin, Pyridin, Picolin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen -20 °C und +100 °C, vorzugsweise bei Temperaturen zwischen 0 °C und +80 °C.

Zur Durchführung des erfindungsgemäßen Verfahrens (a) worden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Die beim erfindungsgemäßen Verfahren (b) als Ausgangsstoffe benötigten Carbonsäuren sind durch die Formel (IV) allgemein definiert.

In Formel (IV) hat R¹ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R¹ angegeben wurde.

Als Beispiele für die Ausgangsstoffe der Formel (IV) seien genannt:
5-Methyl-, 5-Ethyl-, 5-Propyl-, 5-Isopropyl-, 5-Butyl-, 5-Isobutyl-, 5-sec-Butyl- und 5-tert-Butyl-isoxazol-3-carbonsäure, 2-Methyl-, 2-Ethyl-, 2-Propyl-, 2-Isopropyl-, 2-Butyl-, 2-Isobutyl-, 2-sec-Butyl- und 2-tert-Butyl-oxazol-4-carbonsäure, 5-Methyl-, 5-Ethyl-, 5-Propyl-, 5-Isopropyl-, 5-Butyl-, 5-Isobutyl-, 5-sec-Butyl- und 5-tert-Butyl-1,2,4-oxadiazol-3-carbonsäure, 2-Chlor, 2-Methyl-, 2-Ethyl-, 2-Propyl-, 2-Isopropyl-, 2-Butyl-, 2-Isobutyl-, 2-sec-Butyl, 2-tert-Butyl-, 2-Trifluormethyl-, 2-Methoxy-, 2-Ethoxy-, 2-Propoxy-, und 2-Isopropoxy-thiazol-5-carbonsäure, 2,4-Dichlor-thiazol-5-carbonsäure, 2-Methyl-4-chlor-thiazol-5-carbonsäure, 2-Methoxy-4-chlor-thiazol-5-carbonsäure, 4-Methyl-, 4-Ethyl-, 4-Propyl-, 4-Isopropyl-, 4-Butyl-, 4-Isobutyl-, 4-sec-Butyl- und 4-tert-Butyl-thiazol-2-carbonsäure, 5-Methyl-, 5-Ethyl-, 5-Propyl-, 5-Isopropyl-, 5-Butyl-, 5-Isobutyl-, 5-sec-Butyl- und 5-tert-Butyl-thiazol-2-carbonsäure.

Die Carbonsäuren der Formel (IV) sind bekannt und/oder können nach an sich bekannten Verfahren hergestellt werden (vgl. GB-P 1245517; DE-OS 2940189; DE-OS 3013908; Helv. Chim. Acta 27 (1944), 1437-1438; Chem. Ber. 73 (1940), 1240-1252; J. Chem. Soc. 1946, 87-91; loc. cit. 1947, 96-102; Helv. Chim. Acta 29 (1946), 1957-1959; J. Prakt. Chem. 314 (1972), 447-454; Chem. Ber. 94 (1961), 757-761).

Anstatt der Carbonsäuren der Formel (IV) werden beim erfindungsgemäßen Verfahren (b) gegebenenfalls die davon abgeleiteten Carbonsäurechloride oder davon abgeleitete Alkylester (vorzugsweise Methylester oder Ethylester), Aralkylester (vorzugsweise Benzylester) oder Arylester (vorzugsweise Phenylester, in der Phenylgruppe gegebenenfalls durch Cyano, Nitro, Chlor, Fluor, Brom und/oder Methyl substituiert) als Ausgangsstoffe ("reaktionsfähige Derivate") eingesetzt.

Man erhält die Carbonsäurechloride aus den entsprechenden Carbonsäuren nach üblichen Methoden, beispielsweise durch Umsetzung mit üblichen "Chlorierungsmitteln" wie z.B. Phosgen, Oxalylchlorid oder Thionylchlorid, gegebenenfalls in Gegenwart von Reaktionshilfsmitteln, wie z.B. Pyridin, und gegebenenfalls in Gegenwart von Verdünnungsmitteln, wie z.B. Chloroform oder Tetrachlormethan, bei Temperaturen zwischen 0° C und 100° C.

Die entsprechenden Ester der Formel (IV) können aus den entsprechenden Carbonsäurechloriden und geeigneten Alkoholen oder Phenolen nach üblichen Methoden, im allgemeinen durch Umsetzung in Gegenwart eines Säureakzeptors, wie z.B. Triethylamin oder Pyridin, und in Gegenwart eines Verdünnungsmittels, wie z.B. Acetonitril, bei Temperaturen zwischen 0° C und 100° C erhalten werden.

Man kann die genannten Ester jedoch auch direkt aus den Carbonsäuren der Formel (IV) in Gegenwart von Kondensationshilfsmitteln, wie z.B. Dicyclohexylcarbodiimid, gegebenenfalls in Gegenwart von Reaktionshilfsmitteln, wie z.B. 4-Dimethylamino-pyridin, und gegebenenfalls in Gegenwart von Verdünnungsmitteln, wie z.B. Methylenchlorid oder Chloroform, bei Temperaturen zwischen 0° C und 100° C erhalten.

Die beim erfindungsgemäßen Verfahren (b) weiter als Ausgangsstoffe benötigten Aminosulfonylverbindungen sind durch die Formel (V) allgemein definiert.

In Formel (V) haben n, A und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, A und R² angegeben wurden.

Als Beispiele für die Ausgangsstoffe der Formel (V) seien genannt:
Benzolsulfonsäureamid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Difluormethoxy, 2-Trifluormethoxy-, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonyl-benzolsulfonsäureamid sowie (2-Chlor-phenyl)-, (2-Cyano-phenyl)-, (2-Methoxycarbonyl-phenyl)- und (2-Trifluormethoxy-phenyl)-methansulfonsäureamid, 2-Chlor-6-methyl-benzolsulfonsäurechlorid und 2,6-Dichlor-benzolsulfonsäureamid.

Die Aminosulfonylverbindungen der Formel (V) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-A 23 140, 23 141, 23 422, 35 893, 48 143, 51 466, 64 322, 70 041, 44 808 und 44 809; US-P 2 929 820, 4 282 242; 4 348 220 und 4 372 778 sowie Angew. Chem. 93 (1981), 151).

Anstatt der Aminosulfonylverbindungen der Formel (V) werden beim erfindungsgemäßen Verfahren (b) gegebenenfalls die davon abgeleiteten Sulfonylisocyanate ("reaktionsfähige Derivate") als Ausgangsstoffe eingesetzt. Diese Verbindungen sind bekannt und/oder können nach an sich bekannten Verfahren hergestellt werden (vgl. J. Org. Chem. 31 (1966), 2658-2661; EP-A 7687; EP-A 46626; EP-A 21641; EP-A 23140; EP-A 23141; EP-A 70041; EP-A 23422, EP-A 64322; EP-A 34431; EP-A 35893; EP-A 51466, EP-A 44808; EP-A 173312; DE-OS 3132944; EP-A 87780; EP-A 271780).

Das erfindungsgemäße Verfahren (b) zur Herstellung der neuen Verbindungen der Formel (I) wird vorzugsweise unter Verwendung von Verdünnungsmitteln durchgeführt. Es kommen hierbei vorzugsweise die gleichen Verdünnungsmittel in Betracht, die oben für das erfindungsgemäße Verfahren (a) angegeben sind.

Das erfindungsgemäße Verfahren (b) wird gegebenenfalls in Gegenwart eines Reaktionshilfsmittels durchgeführt. Geeignete Reaktionshilfsmittel sind hierbei Verbindungen, mit denen Carbonsäuren in reaktionsfähige Intermediate überführt werden, die dann in situ mit nucleophilen Verbindungen, wie beispielsweise den Sulfonsäureamiden der Formel (V) zu entsprechenden Carbonsäurederivaten umgesetzt werden. Als Beispiele für derartige Reaktionshilfsmittel seien Carbonyldiimidazol und 2-Chlor-1-methyl-pyridiniumiodid genannt.

Für die Umsetzung der Carbonsäurechloride und Ester gemäß Formel (IV) mit Aminosulfonylverbindungen der Formel (V) sind auch Säureakzeptoren, wie sie oben für das erfindungsgemäße Verfahren (a) angegeben sind, als Reaktionshilfsmittel geeignet.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel gegebenenfalls in Gegenwart eines Reaktionshilfsmittels durchgeführt, und das Reaktionsgemisch wird mehrere Stunden bei der jeweils erforderlichen Temperatur gerührt. Die Aufarbeitung erfolgt nach üblichen Methoden.

Aus den erfindungsgemäßen Verbindungen der allgemeinen Formel (I) können gegebenenfalls Salze hergestellt werden. Man erhält solche Salze in einfacher weise nach üblichen Salzbildungsmethoden, beispielsweise durch Lösen oder Dispergieren einer Verbindung der Formel (I) in einem geeigneten Lösungsmittel, wie z. B. Wasser, Methanol, Ethanol, Methylenchlorid oder Aceton, und Zugabe einer geeigneten Säure bzw. Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind, Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die Verbindungen der Formel (I) eignen sich insbesondere zur selektiven Bekämpfung von dikotylen Unkräutern in monokotylen Kulturen, wie z.B. in Mais und Weizen, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); N-Phosphonomethyl-glycin (GLYPHOSATE); Methyl-2-[4,5-dihydro-4-methyl-4-(1-methylethyl)-5-oxo-1H-imidazol-2- yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

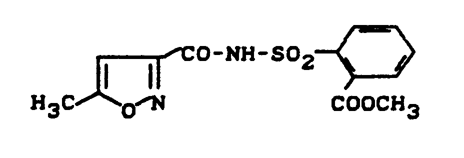

(Verfahren (a))

2,52 g (0,02 Mol) 5-Methyl-isoxazol-3-carboxamid werden in 50 ml 1,4-Dioxan gelöst und nach Zugabe von 3,4 g (0,06 Mol) gepulvertem Kaliumhydroxid 30 Minuten bei 80° C gerührt. Die Mischung wird abgekühlt, bei Raumtemperatur (20° C) mit 5,7 g (0,022 Mol) 2-Chlorsulfonylbenzoesäuremethylester versetzt und anschließend 20 Stunden bei Raumtemperatur gerührt. Danach wird das Lösungsmittel im Vakuum abdestilliert, der Rückstand in Wasser aufgenommen und filtriert. Aus dem Filtrat fällt beim Ansäuern mit Salzsäure das Produkt aus, das auf einer Nutsche gesammelt und auf Ton getrocknet wird.

Man erhält 1,8 g (27% der Theorie) N-(2-Methoxycarbonylphenylsulfonyl)-5-methyl-isoxazol-3-carboxamid vom Schmelzpunkt 101°C.

### Beispiel 2

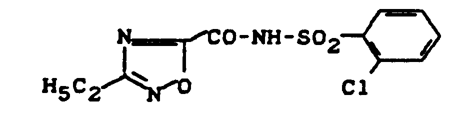

(Verfahren (b))

3,8 g (0,02 Mol) 2-Chlorbenzolsulfonsäureamid werden in das Natriumsalz überführt, indem man es in 100 ml Methanol löst, 1,1 g (0,02 Mol) Natriummethanolat zufügt, 10 Minuten nachrührt und zur Trockene eindampft. Das Natriumsalz wird in 100 ml Acetonitril mit 3,4 g (0,02 Mol) 3-Ethyl-1,2,4-oxadiazol-5-carbonsäureethylester 12 Stunden unter Rückfluß erhitzt. Der nach dem Abdestillieren des Lösungsmittels verbleibende Rückstand wird mit 100 ml 10%iger Kaliumcarbonatlösung verrührt, abgesaugt, mit 1N-Salzsäure und Wasser gewaschen und im Exsikkator getrocknet.

Man erhält 4,6 g (73% der Theorie) N-(2-(Chlorphenylsulfonyl)-3-ethyl-1,2,4-oxadiazol-5-carbonsäureamid vom Schmelzpunkt 142° C.

Analog den Beispielen 1 oder 2 und entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

R¹-CO-NH-SO₂-(A)ₙ-R² (I)

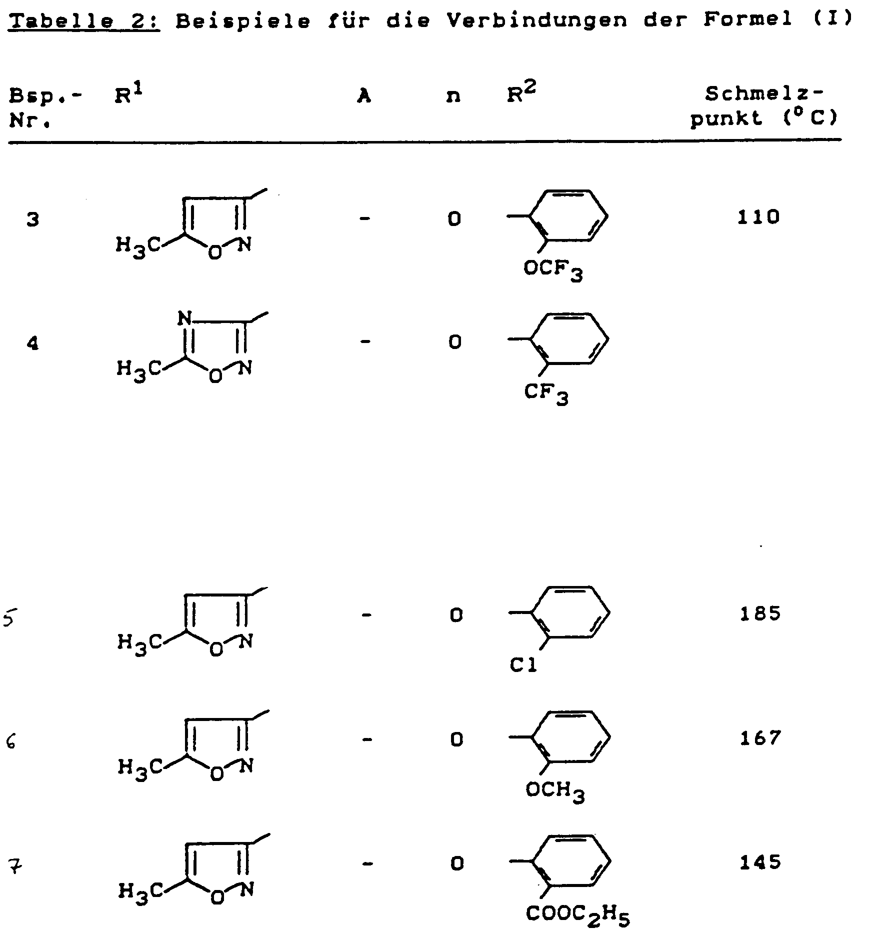

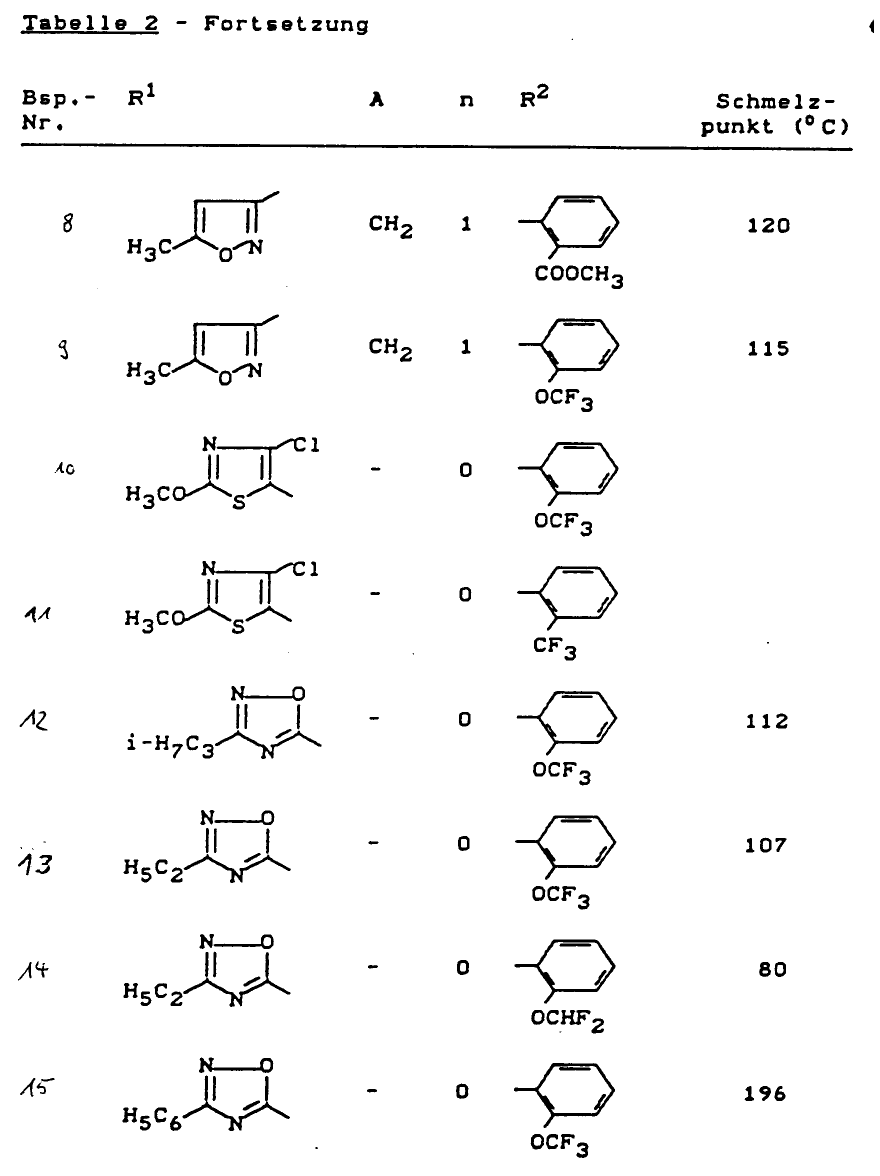

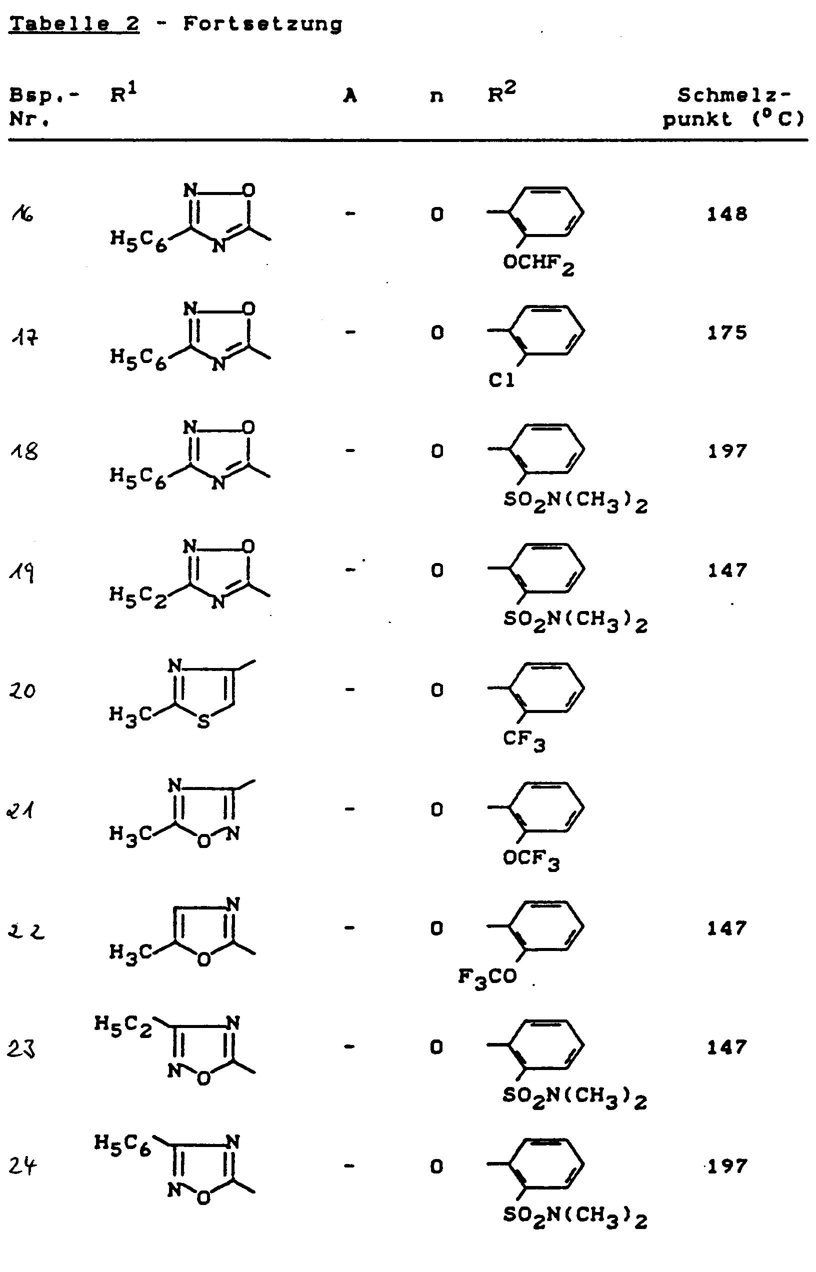

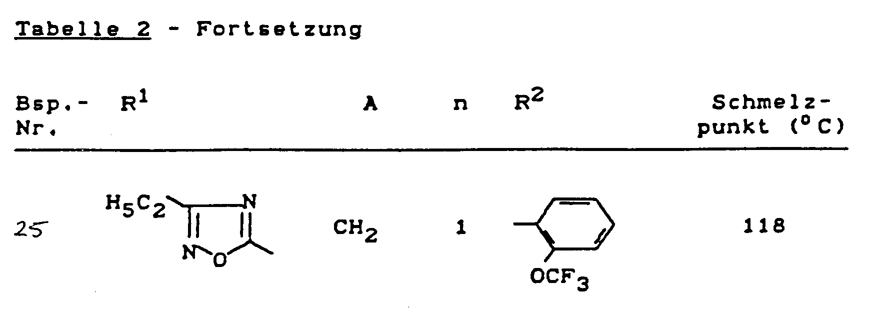

### Beispiel 26

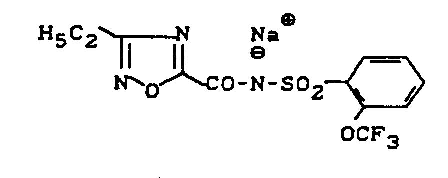

N-(2-Trifluormethoxy-phenylsulfonyl)-3-ethyl-1,2,4-oxadiazol-5-carboxamid-Natriumsalz; Schmelzpunkt: 228°C.

### Beispiel 27

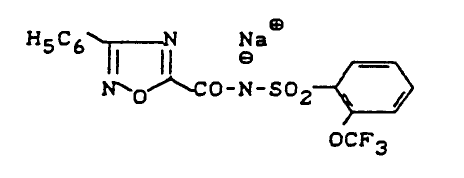

N-(2-Trifluormethoxy-phenylsulfonyl)-3-phenyl-1,2,4-oxadiazol-5-carboximid-Natriumsalz; Schmelzpunkt: 275°C.

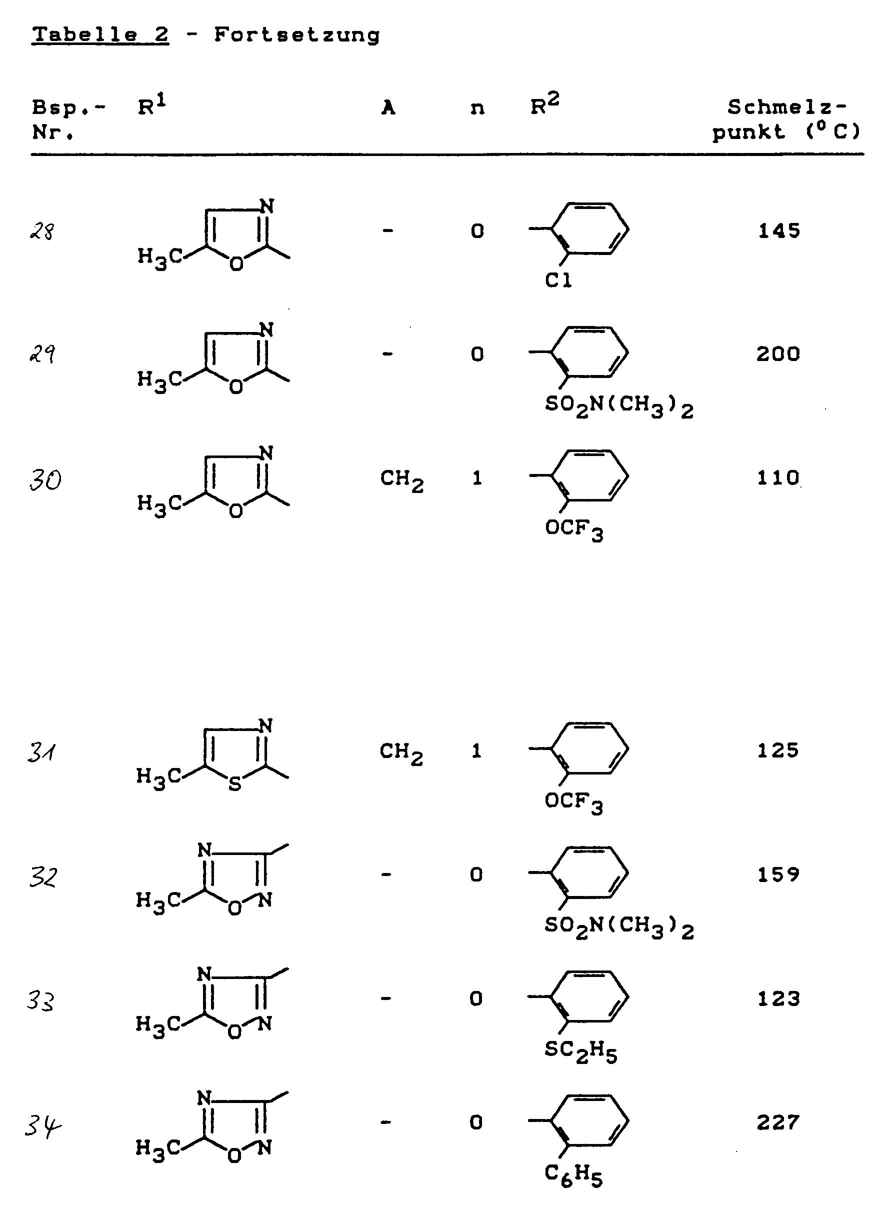

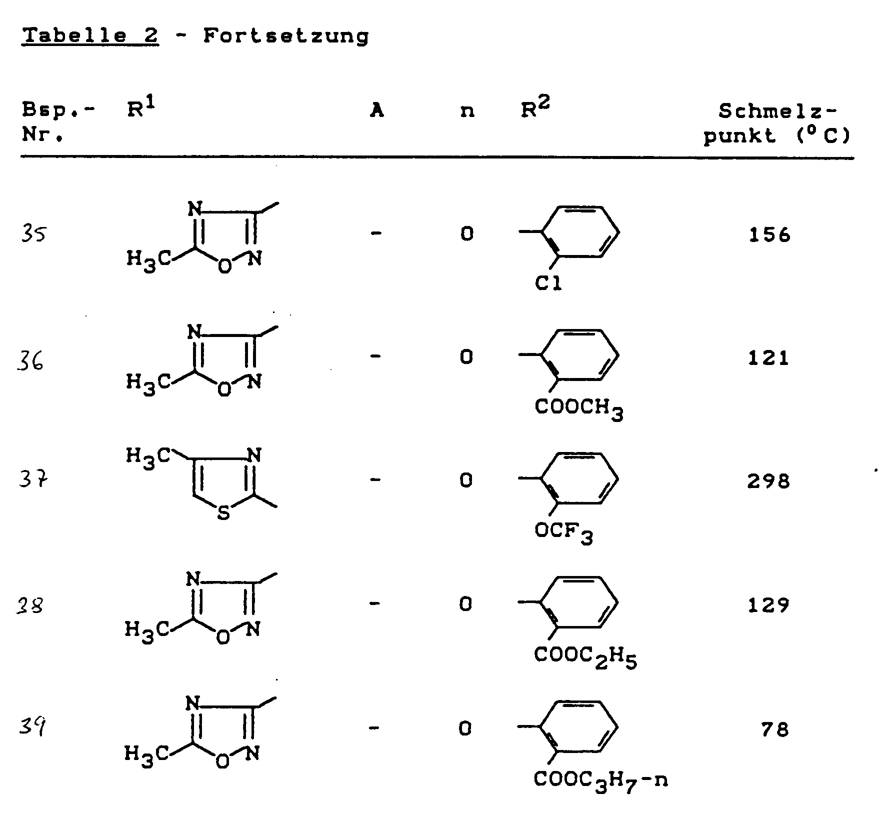

### Anwendungsbeispiele

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung
Eine sehr gute herbizide Wirksamkeit bei guter Verträglichkeit mit den Nutzpflanzen zeigt in diesem Test die Verbindung gemäß Herstellungsbeispiel 1.

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung
Eine sehr gute herbizide Wirkung bei ausgezeichneter Nutzpflanzenselektivität zeigt in diesem Test die Verbindung gemäß Herstellungsbeispiel 1.

## Patentansprüche

1. Sulfonylierte Carbonsäureamide der Formel (I)
R¹-CO-NH-SO₂-(A)ₙ-R² (I)
in welcher
n für die Zahl 0 steht. Falls n für die Zahl 1 steht, steht A insbesondere für CH₂,
A für Sauerstoff, Imino (NH) oder Methylen (CH₂) steht,
R¹ für einen fünfgliedrigen Heteroarylrest aus der Reihe Oxazolyl, Isoxazolyl, Thiazolyl, Isothiazolyl, Oxadiazolyl, oder Thiadiazolyl steht, welcher gegebenenfalls einfach oder mehrfach, gleich oder verschieden durch Halogen oder durch gegebenenfalls halogensubstituiertes C₁-C₆-Alkyl, Phenyl, C₁-C₆-Alkoxy, C₁-C₆-Alkylthio, C₁-C₆-Alkylsulfinyl, C₁-C₆-Alkylsulfonyl, C₁-C₆-Alkylamino oder Di-(C₁-C₄-alkyl)-amino substituiert ist, und
R² für den Rest 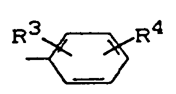 steht, wobei
R³ für Wasserstoff, Fluor, Chlor, Brom, Iod, Cyano, Nitro, Carboxy oder C₁-C₆-Alkyl stehen, welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist, weiterhin R³ und R⁴ unabhängig voneinander für C₂-C₆-Alkenyl stehen, welches gegebenenfalls durch Fluor, Chlor, Brom, Cyano, C₁-C₄-Alkoxy-carbonyl, Carboxy oder Phenyl substituiert ist, ferner R³ und R⁴ unabhängig voneinander für C₂-C₆-Alkinyl, welches gegebenenfalls durch Fluor, Chlor, Brom, Cyano, C₁-C₄-Alkoxy-carbonyl, Carboxy oder Phenyl substituiert ist; für C₁-C₄-Alkoxy, welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist; für C₁-C₄-Alkylthio, welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist; für C₃-C₆-Alkenyloxy, welches gegebenenfalls durch Fluor, Chlor, Brom, Cyano oder C₁-C₄-Alkoxy-carbonyl substituiert ist, für C₂-C₆-Alkenylthio, welches gegebenenfalls durch Fluor, Chlor, Brom, Cyano, Nitro, C₁-C₃-Alkylthio oder C₁-C₄-Alkoxycarbonyl substituiert ist; C₃-C₆-Alkinyloxy, C₃-C₆-Alkinylthio oder für den Rest -S(O)ₚ-R⁵ steht ,
R³ weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁶ steht , R³ weiterhin für C₁-C₄-Alkylsulfonyloxy, Di-(C₁-C₄-alkyl)-aminosulfonylamino oder für den Rest -CH=N-R⁷ steht , wobei
p für die Zahlen 1 oder 2 steht und
R⁵ für C₁-C₄-Alkyl steht, welches gegebenenfalls durch Fluor, Chlor, Brom, Cyano oder C₁-C₄-Alkoxy-carbonyl substituiert ist oder R⁵ für C₁-C₄-Alkoxy, C₁-C₄-Alkoxy-C₁-C₄-alkylamino oder Di-(C₁-C₄-alkyl)-amino steht,
R⁶ für C₁-C₆-Alkyl, C₁-C₆-Alkoxy, welches gegebenenfalls durch Fluor, Chlor, Methoxy oder Ethoxy substituiert ist; C₃-C₆-Cycloalkoxy, C₃-C₆-Alkenyloxy, C₁-C₄-Alkylthio, Amino, C₁-C₄-Alkylamino, C₁-C₄-Alkoxyamino, C₁-C₄-Alkoxy-C₁-C₄-alkyl-amino oder Di-(C₁-C₄-alkyl)-amino steht, welche gegebenenfalls durch Fluor und/oder Chlor substituiert sind und
R⁷ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/ oder Chlor substituiertes C₁-C₆-Alkoxy, C₃-C₆-Alkenyloxy, C₃-C₆-Alkinyloxy oder Benzyloxy, R⁷ weiterhin für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht und
R⁴ für Wasserstoff steht, sowie Salze von Verbindungen der Formel (I), wobei R³ in ortho-Position steht .

2. Sulfonylierte Carbonsäureamide der Formel (I) gemäß Anspruch 1, in welcher
n für die Zahl 0 steht. Falls n für die Zahl 1 steht, steht A insbesondere für CH₂,
A für Sauerstoff, Imino (NH) oder Methylen (CH₂) steht,
R¹ für einen fünfgliedrigen Heteroarylrest aus der Reihe Oxazolyl, Isoxazolyl, Thiazolyl, 1,2,4-Oxadiazolyl, 1,3,4-Oxadiazolyl, 1,2,4-Thiadiazolyl, 1,3,4-Thiadiazolyl steht, welcher gegebenenfalls durch Fluor, Chlor, Brom, Methyl, Ethyl, Propyl, Isopropyl, Butyl, Isobutyl, sec-Butyl, tert-Butyl, Fluormethyl, Chlormethyl, Difluormethyl, Dichlormethyl, Trifluormethyl, Trichlormethyl, Chlorfluormethyl, Chlordifluormethyl, Fluordichlormethyl, Phenyl, Methoxy, Ethoxy, Propoxy, Isopropyloxy, Difluormethoxy, Trifluormethoxy, Chlordifluormethoxy, Methylthio, Ethylthio, Propylthio, Isopropylthio, Difluormethylthio, Trifluormethylthio, Chlordifluormethylthio, Methylsulfinyl, Ethylsulfinyl, Propylsulfinyl, Methylsulfonyl, Ethylsulfonyl, Propylsulfonyl, Methylamino, Ethylamino, Propylamino, Isopropylamino und/oder Dimethylamino substituiert ist, und
R² für den Rest 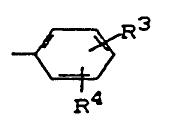 steht, worin
R³ für Wasserstoff, Fluor, Chlor, Brom, Cyano, Nitro, Carboxy, Methyl, Trifluormethyl, Methoxy, Ethoxy, 2-Chlor-ethoxy, Difluormethoxy, Trifluormethoxy, C₁-C₃-Alkylthio, C₁-C₃-Alkyl-sulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
R⁴ für Wasserstoff steht; wobei R³ in ortho-Position steht ,
sowie Salze von Verbindungen der Formel (I) mit Basen.

3. Verbindungen der Formel (I) gemäß Anspruch 1 oder 2 mit Basen, wie Natrium-Kalium- oder Calcium-hydroxid, -hydrid, -amid oder -carbonat, Natrium- oder Kalium-C₁-C₄-alkanolaten, Ammoniak, C₁-C₄-Alkylaminen, Di-(C₁-C₄-alkyl)-aminen oder Tri-(C₁-C₄-alkyl)-aminen.

4. Verfahren zur Herstellung von sulfonylierten Carbonsäureamiden der Formel (I)
R¹-CO-NH-SO₂-(A)ₙ-R² (I)
in welcher n, A, R¹ und R² die in Anspruch 1 genannten Bedeutungen haben, dadurch gekennzeichnet, daß man
(a) Carbonsäureamide der allgemeinen Formel (II)
R¹-CO-NH₂ (II)
in welcher
R¹ die oben angegebene Bedeutung hat,
mit Sulfonylierungsmitteln der allgemeinen Formel (III)
X-SO₂-(A)ₙ-R² (III)
in welcher
n, A und R² die oben angegebene Bedeutung haben und
X für Halogen steht,
gegebenenfalls in Gegenwart eines Säureakzeptors und gegebenenfalls in Gegenwart eines Verdünnungsmittels umsetzt, oder daß man
(b) Carbonsäuren der allgemeinen Formel (IV)
R¹-COOH (IV)
in welcher
R¹ die oben angegebene Bedeutung hat,
oder reaktionsfähige Derivate der Carbonsäuren der Formel (IV)
mit Aminosulfonylverbindungen der allgemeinen Formel (V)
H₂N-SO₂-(A)ₙ-R² (V)
in welcher
n, A und R² die oben angegebene Bedeutung haben,
oder mit reaktionsfähigen Derivaten der Verbindungen der Formel (V)
gegebenenfalls in Gegenwart von Reaktionshilfsmitteln und gegebenenfalls in Gegenwart von Verdünnungsmitteln umsetzt und gegebenenfalls die nach Verfahren (a) oder (b) erhaltenen Produkte in Salze überführt.

5. Herbizide Mittel, gekennzeichnet durch einen Gehalt an mindestens einem sulfonylierten Carbonsäureamid der Formel (I) gemäß den Ansprüchen 1 oder 4.

6. Verfahren zur Bekämpfung von unerwünschten Pflanzen, dadurch gekennzeichnet, daß man sulfonylierte Carbonsäureamide der Formel (I) gemäß den Ansprüchen 1 oder 4 auf die Pflanzen und/oder ihren Lebensraum einwirken läßt.

7. Verwendung von sulfonylierten Carbonsäureamiden der Formel (I) gemäß den Ansprüchen 1 oder 4 zur Bekämpfung von unerwünschten Pflanzen.

8. Verfahren zur Herstellung von herbiziden Mitteln, dadurch gekennzeichnet, daß man sulfonylierte Carbonsäureamide der Formel (I) gemäß den Ansprüchen 1 oder 4 mit Streckmitteln und/oder oberflächenaktiven Substanzen vermischt.

## Claims

1. Sulphonylated carboxamides of the formula (I)
R¹-CO-NH-SO₂-(A)ₙ-R² (I)
in which
n represents the number 0. If n represents the number 1, A in particular represents CH₂,
A represents oxygen, imino (NH) or methylene (CH₂),
R¹ represents a five-membered heteroaryl radical from the series comprising oxazolyl, isoxazolyl, thiazolyl, isothiazolyl, oxadiazolyl and thiadiazolyl, which is optionally monosubstituted or polysubstituted by identical or different halogen or by optionally halogen-substituted C₁-C₆-alkyl, phenyl, C₁-C₆-alkoxy, C₁-C₆-alkylthio, C₁-C₆-alkylsulphinyl, C₁-C₆-alkylsulphonyl, C₁-C₆-alkylamino or di-(C₁-C₄-alkyl)-amino, and
R² represents the radical 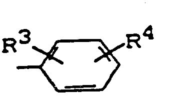 in which
R³ represents hydrogen, fluorine, chlorine, bromine, iodine, cyano, nitro, carboxyl or C₁-C₆-alkyl which is optionally substituted by fluorine, chlorine, bromine, cyano, carboxyl, C₁-C₄-alkoxycarbonyl, C₁-C₄-alkylamino-carbonyl, di-(C₁-C₄-alkyl)amino-carbonyl, hydroxyl, C₁-C₄-alkoxy, formyloxy, C₁-C₄-alkyl-carbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)-aminosulphonyl, C₃-C₆-cycloalkyl or phenyl, R³ and R⁴ furthermore independently of one another represent C₂-C₆-alkenyl which is optionally substituted by fluorine, chlorine, bromine, cyano, C₁-C₄-alkoxycarbonyl, carboxyl or phenyl, R³ and R⁴ additionally independently of one another represents C₂-C₆-alkinyl which is optionally substituted by fluorine, chlorine, bromine, cyano, C₁-C₄-alkoxy-carbonyl, carboxyl or phenyl; C₁-C₄-alkoxy which is optionally substituted by fluorine, chlorine, bromine, cyano, carboxyl, C₁-C₄-alkoxycarbonyl, C₁-C₄-alkoxy, C₁-C₄-alkylthio, C₁-C₄-alkylsulphinyl or C₁-C₄-alkylsulphonyl; C₁-C₄-alkylthio which is optionally substituted by fluorine, chlorine, bromine, cyano, carboxyl, C₁-C₄-alkoxy-carbonyl, C₁-C₄-alkylthio, C₁-C₄-alkylsulphinyl or C₁-C₄-alkylsulphonyl; C₃-c₆-alkenyloxy which is optionally substituted by fluorine, chlorine, bromine, cyano or C₁-C₄-alkoxy-carbonyl, C₂-C₆-alkenylthio which is optionally substituted by fluorine, chlorine, bromine, cyano, nitro, C₁-C₃-alkylthio or C₁-C₄-alkoxycarbonyl; C₃-C₆-alkinyloxy, C₃-C₆-alkinylthio or the radical -S(O)ₚ-R⁵,
R³ furthermore represents phenyl or phenoxy, C₁-C₄-alkylcarbonylamino, C₁-C₄-alkoxycarbonylamino, C₁-C₄-alkylamino-carbonylamino, di-(C₁-C₄-alkyl)-amino-carbonylamino, or the radical -CO-R⁶, R³ furthermore represents C₁-C₄-alkylsulphonyloxy, di-(C₁-C₄-alkyl)-aminosulphonylamino or the radical -CH=N-R⁷, in which
p represents the numbers 1 or 2 and
R⁵ represents C₁-C₄-alkyl which is optionally substituted by fluorine, chlorine, bromine, cyano or C₁-C₄-alkoxy-carbonyl or R⁵ represents C₁-C₄-alkoxy, C₁-C₄-alkoxy-C₁-C₄-alkylamino or di-(C₁-C₄-alkyl)-amino,
R⁶ represents C₁-C₆-alkyl, C₁-C₆-alkoxy which is optionally substituted by fluorine, chlorine, methoxy or ethoxy; C₃-C₆-cycloalkoxy, C₃-C₆-alkenyloxy, C₁-C₄-alkylthio, amino, C₁-C₄-alkylamino, C₁-C₄-alkoxyamino, C₁-C₄-alkoxy-C₁-C₄-alkyl-amino or di-(C₁-C₄-alkyl)-amino which are optionally substituted by fluorine and/or chlorine and
R⁷ represents C₁-C₆-alkyl which is optionally substituted by fluorine, chlorine, cyano, carboxyl, C₁-C₄-alkoxy, C₁-C₄-alkylthio, C₁-C₄-alkylsulphinyl or C₁-C₄-alkylsulphonyl, benzyl which is optionally substituted by fluorine or chlorine, C₃-C₆-alkenyl or C₃-C₆-alkinyl which are optionally substituted by fluorine or chlorine, phenyl which is optionally substituted by fluorine, chlorine, bromine, C₁-C₄-alkyl, C₁-C₄-alkoxy, trifluoromethyl, trifluoromethoxy or trifluoromethylthio, C₁-C₆-alkoxy, C₃-C₆-alkenyloxy, C₃-C₆-alkinyloxy or benzyloxy which are optionally substituted by fluorine and/or chlorine, R⁷ furthermore represents amino, C₁-C₄-alkylamino, di-(C₁-C₄-alkyl)-amino, phenylamino, C₁-C₄-alkyl-carbonylamino, C₁-C₄-alkoxy-carbonylamino, C₁-C₄-alkyl-sulphonylamino or phenyl sulphonylamino which is optionally substituted by fluorine, chlorine, bromine or methyl, and
R⁴ represents hydrogen,
and salts of compounds of the formula (I),
where R³ is in the ortho position.

2. Sulphonylated carboxamides of the formula (I) according to Claim 1, in which
n represents the number 0. If n represents the number 1, A in particular represents CH₂,
A represents oxygen, imino (NH) or methylene (CH₂),
R¹ represents a five-membered heteroaryl radical from the series comprising oxazolyl, isoxazolyl, thiazolyl, 1,2,4-oxadiazolyl, 1,3,4-oxadiazolyl, 1,2,4-thiadiazolyl and 1,3,4-thiadiazolyl, which is optionally substituted by fluorine, chlorine, bromine, methyl, ethyl, propyl, isopropyl, butyl, isobutyl, sec-butyl, tert-butyl, fluoromethyl, chloromethyl, difluoromethyl, dichloromethyl, trifluoromethyl, trichloromethyl, chlorofluoromethyl, chlorodifluoromethyl, fluorodichloromethyl, phenyl, methoxy, ethoxy, propoxy, isopropyloxy, difluoromethoxy, trifluoromethoxy, chlorodifluoromethoxy, methylthio, ethylthio, propylthio, isopropylthio, difluoromethylthio, trifluoromethylthio, chlorodifluoromethylthio, methylsulphinyl, ethylsulphinyl, propylsulphinyl, methylsulphonyl, ethylsulphonyl, propylsulphonyl, methylamino, ethylamino, propylamino, isopropylamino and/or dimethylamino, and
R² represents the radical 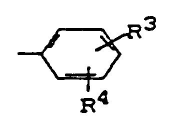 in which
R³ represents hydrogen, fluorine, chlorine, bromine, cyano, nitro, carboxyl, methyl, trifluoromethyl, methoxy, ethoxy, 2-chloroethoxy, difluoromethoxy, trifluoromethoxy, C₁-C₃-alkylthio, C₁-C₃-alkyl-sulphinyl, C₁-C₃-alkylsulphonyl, dimethylaminosulphonyl, diethylaminosulphonyl, N-methoxy-N-methylaminosulphonyl, phenyl, phenoxy or C₁-C₃-alkoxycarbonyl and
R⁴ represents hydrogen;
where R³ is in the ortho position,
and salts of compounds of the formula (I) with bases.

3. Compounds of the formula (I) according to Claim 1 or 2 with bases, such as the hydroxide, hydride, amide or carbonate of sodium, potassium or calcium, the C₁-C₄-alkoxides of sodium or potassium, ammonia, C₁-C₄-alkylamines, di-(C₁-C₄-alkyl)-amines or tri-(C₁-C₄-alkyl)-amines.

4. Process for the preparation of sulphonylated carboxamides of the formula (I)
R¹-CO-NH-SO₂-(A)ₙ-R² (I)
in which n, A, R¹ and R² have the meanings mentioned in Claim 1,
characterized in that
(a) carboxamides of the general formula (II)
R¹-CO-NH₂ (II)
in which
R¹ has the abovementioned meaning,
are reacted with sulphonylating agents of the general formula (III)
X-SO₂-(A)ₙ-R² (III)
in which
n, A and R² have the abovementioned meaning and
X represents halogen,
if appropriate in the presence of an acid acceptor and if appropriate in the presence of a diluent, or in that
(b) carboxylic acids of the general formula (IV)
R¹-COOH (IV)
in which
R¹ has the abovementioned meaning,
or reactive derivatives of carboxylic acids of the formula (IV)
are reacted with aminosulphonyl compounds of the general formula (V)
H₂N-SO₂-(A)ₙ-R² (V)
in which
n, A and R² have the abovementioned meaning,
or with reactive derivatives of the compounds of the formula (V)
if appropriate in the presence of reaction auxiliaries and if appropriate in the presence of diluents and, if appropriate, the products obtained by processes (a) or (b) are converted into salts.

5. Herbicidal agents, characterized in that they contain at least one sulphonylated carboxamide of the formula (I) according to Claims 1 or 4.

6. Process for combating undesired plants, characterized in that sulphonylated carboxamides of the formula (I) according to Claims 1 or 4 are allowed to act on the plants and/or their environment.

7. Use of sulphonylated carboxamides of the formula (I) according to Claims 1 or 4 for combating undesired plants.

8. Process for the production of herbicidal agents, characterized in that sulphonylated carboxamides of the formula (I) according to Claims 1 or 4 are mixed with extenders and/or surface-active substances.

## Revendications

1. Amides d'acides carboxyliques sulfonylés répondant à la formule (I)
R¹-CO-NH-SO₂-(A)ₙ-R² (I)
dans laquelle
n représente le nombre 0. Au cas où n représente le nombre 1, A représente en particulier CH₂,
A représente un atome d'oxygène, un groupe imino (NH) ou un groupe méthylène (CH₂),
R¹ représente un radical hétéroaryle pentagonal choisi parmi la série comprenant un radical oxalyle, un radical isoxazolyle, un radical thiazolyle, un radical isothiazolyle, un radical oxadiazolyle ou un radical thiadiazolyle, qui porte éventuellement un ou plusieurs substituants identiques ou différents halogéno ou alkyle en C₁-C₆ (portant éventuellement un ou plusieurs substituants identiques ou différents halogéno), phényle, alcoxy en C₁-C₆, alkyl(en C₁-C₆)thio, alkyl(en C₁-C₆)sulfinyle, alkyl(en C₁-C₆)sulfonyle, alkyl(en C₁-C₆)amino ou di(alkyl en C₁-C₄)amino, et
R² représente le radical 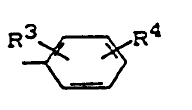 dans lequel
R³ représente un atome d'hydrogène, un atome de fluor, un atome de chlore, un atome de brome, un atome d'iode, un groupe cyano, un groupe nitro, un groupe carboxyle ou un groupe alkyle en C₁-C₆ qui porte éventuellement un ou plusieurs substituants identiques ou différents fluoro, chloro, bromo, cyano, carboxyle, alcoxy(en C₁-C₄)carbonyle, alkyl(en C₁-C₄)aminocarbonyle, di(alkyl en C₁-C₄)aminocarbonyle, hydroxyle, alcoxy(en C₁-C₄), formyloxy, alkyl(en C₁-C₄)carbonyloxy, alcoxy(en C₁-C₄)carbonyloxy, alkyl(en C₁-C₄)amino-carbonyloxy, alkyl(en C₁-C₄)thio, alkyl(en C₁-C₄)sulfinyle, alkyl(en C₁-C₄)sulfonyle, di(alkyl en C₁-C₄)aminosulfonyle, cycloalkyle en C₃-C₆ ou phényle; en outre, R³ et R⁴ représentent, indépendamment l'un de l'autre, un groupe alcényle en C₂-C₆ qui porte éventuellement un ou plusieurs substituants identiques ou différents fluoro, chloro, bromo, cyano, alcoxy(en C₁-C₄)carbonyle, carboxyle ou phényle; en outre, R³ et R⁴ représentent, indépendamment l'un de l'autre, un groupe alcynyle en C₂-C₆ qui porte éventuellement un ou plusieurs substituants identiques ou différents fluoro, chloro, bromo, cyano, alcoxy(en C₁-C₄)carbonyle, carboxyle ou phényle; un groupe alcoxy en C₁-C₄ qui porte éventuellement un ou plusieurs substituants identiques ou différents fluoro, chloro, bromo, cyano, carboxyle, alcoxy(en C₁-C₄)carbonyle, alcoxy en C₁-C₄, alkyl(en C₁-C₄)thio, alkyl(en C₁-C₄)sulfinyle ou alkyl(en C₁-C₄)sulfonyle; un groupe alkyl(en C₁-C₄)thio qui porte éventuellement un ou plusieurs substituants identiques ou différents fluoro, chloro, bromo, cyano, carboxyle, alcoxy(en C₁-C₄)carbonyle, alkyl(en C₁-C₄)thio, alkyl(en C₁-C₄)sulfinyle ou alkyl(en C₁-C₄)sulfonyle; un groupe alcényl(en C₃-C₆)oxy qui porte éventuellement un ou plusieurs substituants identiques ou différents fluoro, chloro, bromo, cyano ou alcoxy(en C₁-C₄)carbonyle; un groupe alcényl(en C₂-C₆)thio qui porte éventuellement un ou plusieurs substituants identiques ou différents fluoro, chloro, bromo, cyano, nitro, alkyl(en C₁-C₃)thio ou alcoxy(en C₁-C₄)carbonyle; un groupe alcynyl(en C₃-C₆)oxy, un groupe alcynyl(en C₃-C₆)thio ou encore le radical -S(O)ₚ-R⁵,
R³ représente, en outre, un groupe phényle ou un groupe phénoxy; un groupe alkyl(en C₁-C₄)carbonylamino, un groupe alcoxy(en C₁-C₄)carbonylamino, un groupe alkyl(en C₁-C₄)aminocarbonylamino, un groupe di(alkyl en C₁-C₄)aminocarbonylamino ou encore le radical -CO-R⁶; R³ représente, en outre, un groupe alkyl(en C₁-C₄)sulfonyloxy, un groupe di(alkyl en C₁-C₄)aminosulfonylamino ou encore le radical -CH=N-R⁷, où
p représente les nombres 1 ou 2, et
R⁵ représente un groupe alkyle en C₁-C₄ qui porte éventuellement un ou plusieurs substituants identiques ou différents fluoro, chloro, bromo, cyano ou alcoxy(en C₁-C₄)carbonyle, ou bien R⁵ représente un groupe alcoxy en C₁-C₄, un groupe alcoxy(en C₁-C₄)alkyl(en C₁-C₄)amino ou encore un groupe di(alkyl en C₁-C₄)amino,
R⁶ représente un groupe alkyle en C₁-C₆, un groupe alcoxy en C₁-C₆ qui porte éventuellement un ou plusieurs substituants identiques ou différents fluoro, chloro, méthoxy ou éthoxy; un groupe cycloalcoxy en C₃-C₆, un groupe alcényl(en C₃-C₆)oxy, un groupe alkyl(en C₁-C₄)thio, un groupe amino, un groupe alkyl(en C₁-C₄)amino, un groupe alcoxy(en C₁-C₄)amino, un groupe alcoxy(en C₁-C₄)alkyl(en C₁-C₄)amino ou encore un groupe di(alkyl en C₁-C₄)amino, qui portent éventuellement un ou plusieurs substituants identiques ou différents fluoro et/ou chloro, et
R⁷ représente un groupe alkyle en C₁-C₆ portant éventuellement un ou plusieurs substituants identiques ou différents fluoro, chloro, cyano, carboxyle, alcoxy en C₁-C₄, alkyl(en C₁-C₄)thio, alkyl(en C₁-C₄)sulfinyle ou alkyl(en C₁-C₄)sulfonyle; un groupe benzyle portant éventuellement un ou plusieurs substituants identiques ou différents fluoro ou chloro; un groupe alcényle en C₃-C₆ ou un groupe alcynyle en C₃-C₆ portant éventuellement un ou plusieurs substituants identiques ou différents fluoro ou chloro; un groupe phényle portant éventuellement un ou plusieurs substituants identiques ou différents fluoro, chloro, bromo, alkyle en C₁-C₄, alcoxy en C₁-C₄, trifluorométhyle, trifluorométhoxy ou trifluorométhylthio; un groupe alcoxy en C₁-C₆, un groupe alcényl(en C₃-C₆)oxy, un groupe alcynyl(en C₃-C₆)oxy ou un groupe benzyloxy portant éventuellement un ou plusieurs substituants identiques ou différents fluoro et/ou chloro, R⁷ représente en outre un groupe amino, un groupe alkyl(en C₁-C₄)amino, un groupe di(alkyl en C₁-C₄)amino, un groupe phénylamino, un groupe alkyl(en C₁-C₄)carbonylamino, un groupe alcoxy(en C₁-C₄)carbonylamino, un groupe alkyl(en C₁-C₄)sulfonylamino ou encore un groupe phénylsulfonylamino portant éventuellement un ou plusieurs substituants identiques ou différents fluoro, chloro, bromo ou méthyle, et
R⁴ représente un atome d'hydrogène,
ainsi que des sels des composés de formule (I),
R³ se trouvant en position ortho.

2. Amides d'acides carboxyliques sulfonylés de formule (I) selon la revendication 1, dans laquelle
n représente le nombre 0. Au cas où n représente le nombre 1, A représente en particulier CH₂,
A représente un atome d'oxygène, un groupe imino (NH) ou un groupe méthylène (CH₂),
R¹ représente un radical hétéroaryle pentagonal choisi parmi la série comprenant un radical oxazolyle, un radical isoxazolyle, un radical thiazolyle, un radical 1,2,4-oxadiazolyle, un radical 1,3,4-oxadiazolyle, un radical 1,2,4-thiadiazolyle, un radical 1,3,4-thiadiazolyle, qui porte éventuellement un ou plusieurs substituants identiques ou différents fluoro, chloro, bromo, méthyle, éthyle, propyle, isopropyle, butyle, isobutyle, sec.-butyle, tert.-butyle, fluorométhyle, chlorométhyle, difluorométhyle, dichlorométhyle, trifluorométhyle, trichlorométhyle, chlorofluorométhyle, chlorodifluorométhyle, fluorodichlorométhyle, phényle, méthoxy, éthoxy, propoxy, isopropyloxy, difluorométhoxy, trifluorométhoxy, chlorodifluorométhoxy, méthylthio, éthylthio, propylthio, isopropylthio, difluorométhylthio, trifluorométhylthio, chlorodifluorométhylthio, méthylsulfinyle, éthylsulfinyle, propylsulfinyle, méthylsulfonyle, éthylsulfonyle, propylsulfonyle, méthylamino, éthylamino, propylamino, isopropylamino et/ou diméthylamino, et
R² représente le radical 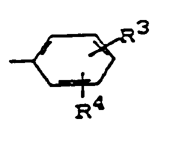 dans lequel
R³ représente un atome d'hydrogène, un atome de fluor, un atome de chlore, un atome de brome, un groupe cyano, un groupe nitro, un groupe carboxyle, un groupe méthyle, un groupe trifluorométhyle, un groupe méthoxy, un groupe éthoxy, un groupe 2-chloréthoxy, un groupe difluorométhoxy, un groupe trifluorométhoxy, un groupe alkyl(en C₁-C₃)thio, un groupe alkyl(en C₁-C₃)sulfinyle, un groupe alkyl(en C₁-C₃)sulfonyle, un groupe diméthylaminosulfonyle, un groupe diéthylaminosulfonyle, un groupe N-méthoxy-N-méthylaminosulfonyle, un groupe phényle, un groupe phénoxy ou un groupe alcoxy(en C₁-C₃)carbonyle, et
R⁴ représente un atome d'hydrogène;
R³ se trouvant en position ortho,
ainsi que des sels des composés de formule (I) avec des bases.

3. Composés de formule (I) selon la revendication 1 ou 2, avec des bases telles que l'hydroxyle, l'hydrure, l'amidure ou le carbonate de sodium, de potassium ou de calcium des alcanolates en C₁-C₄ de sodium ou de potassium, l'ammoniac, des alkyl(en C₁-C₄)amines, des di(alkyl en C₁-C₄)amines ou des tri(alkyl en C₁-C₄)amines.

4. Procédé pour la préparation d'amides d'acides carboxyliques sulfonylés de formule (I)
R¹-CO-NH-SO₂-(A)ₙ-R² (I)
dans laquelle n, A, R¹ et R² ont les significations indiquées à la revendication 1,
caractérisé en ce que
(a) on fait réagir des amides d'acides carboxyliques répondant à la formule générale (II)
R¹-CO-NH₂ (II)
dans laquelle
R¹ a la signification indiquée ci-dessus,
avec des agents de sulfonylation répondant à la formule générale (III)
X-SO₂-(A)ₙ-R² (III)
dans laquelle
n, A et R² ont la signification indiquée ci-dessus et
X représente un atome d'halogène,
éventuellement en présence d'un accepteur d'acides et éventuellement en présence d'un diluant, ou bien en ce que
(b) on fait réagir des acides carboxyliques répondant à la formule générale (IV)
R¹-COOH (IV)
dans laquelle
R¹ a la signification indiquée ci-dessus,
ou encore des dérivés réactifs des acides carboxyliques de formule (IV)
avec des composés aminosulfonyle répondant à la formule générale (V)
H₂N-SO₂-(A)ₙ-R² (V)
dans laquelle
n, A et R² ont la signification indiquée ci-dessus,
ou bien avec des dérivés réactifs des composés de formule (V)
éventuellement en présence d'adjuvants réactionnels et éventuellement en présence de diluants, et on transforme éventuellement en sels les produits obtenus conformément aux procédés (a) ou (b).

5. Agents herbicides caractérisés par une teneur en au moins un amide d'acide carboxylique sulfonylé de formule (I) selon les revendications 1 ou 4.

6. Procédé pour lutter contre des plantes non désirées, caractérisé en ce qu'on laisse agir des amides d'acides carboxyliques sulfonylés de formule (I) selon les revendications 1 ou 4, sur les plantes et/ou sur leur biotope.

7. Utilisation d'amides d'acides carboxyliques sulfonylés de formule (I) selon les revendications 1 ou 4, pour lutter contre des plantes non désirées.

8. Procédé pour la préparation d'agents herbicides, caractérisé en ce qu'on mélange des amides d'acides carboxyliques sulfonylés de formule (I) selon les revendications 1 ou 4, avec des diluants et/ou avec des substances tensioactives.
